(19) Europäisches Patentamt
European Patent Office
Office européen des brevets

(11) Numéro de publication: **0 551 498 B1**

(12) # FASCICULE DE BREVET EUROPEEN

(45) Date de publication du fascicule du brevet: **18.10.95**

(51) Int. Cl.[6]: **A61K 7/06**, A61K 7/48

(21) Numéro de dépôt: **92917491.0**

(22) Date de dépôt: **30.07.92**

(86) Numéro de dépôt internationale :
**PCT/FR92/00746**

(87) Numéro de publication internationale :
**WO 93/02656 (18.02.93 93/05)**

(54) **DISPERSIONS CATIONIOUES A BASE DE CERAMIDES ET/OU DE GLYCOCERAMIDES.**

(30) Priorité: **01.08.91 FR 9109824**

(43) Date de publication de la demande:
**21.07.93 Bulletin 93/29**

(45) Mention de la délivrance du brevet:
**18.10.95 Bulletin 95/42**

(84) Etats contractants désignés:
**AT BE CH DE DK ES GB GR IT LI NL SE**

(56) Documents cités:
**EP-A- 0 260 697**
**EP-A- 0 278 505**
**EP-A- 0 420 722**
**EP-A- 0 446 094**

(73) Titulaire: **L'OREAL**
**14, rue Royale**
**F-75008 Paris (FR)**

(72) Inventeur: **DUBIEF, Claude**
**9, rue Edmond-Rostand**
**F-78150 Le-Chesnay (FR)**
Inventeur: **CAUWET, Danièle**
**53, rue de Charonne**
**F-75011 Paris (FR)**

(74) Mandataire: **Casalonga, Axel**
**BUREAU D.A. CASALONGA - JOSSE**
**Morassistrasse 8**
**D-80469 München (DE)**

EP 0 551 498 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des dispersions cationiques pour le traitement des cheveux ou de la peau, des compositions cosmétiques les renfermant et leurs applications cosmétiques.

Il est bien connu que les cheveux sont sensibilisés ou fragilisés à des degrés divers par l'action des agents atmosphériques, ainsi que par l'action des différents traitements capillaires tels que les permanentes, le défrisage, la teinture, la décoloration. Les cheveux deviennent alors difficiles à démêler et à coiffer. De plus, ils deviennent rêches au toucher.

Pour faciliter le démêlage et améliorer leur douceur au toucher, on utilise couramment des agents tensio-actifs cationiques. Ces agents tensio-actifs ont malheureusement tendance à alourdir la chevelure et à lui donner un aspect gras. Ce phénomène est d'autant plus accentué que les cheveux traités sont fins.

Les céramides ont déjà été proposés dans des compositions capillaires. En raison de leur insolubilité dans les milieux aqueux, ils ont été jusqu'ici souvent mis en oeuvre dans des formulations à base d'agents tensio-actifs anioniques et/ou non-ioniques.

La demanderesse a constaté que les émulsions ou solutions à base de céramides ne permettaient pas d'obtenir de bonnes propriétés de démêlage des cheveux.

La demanderesse vient de découvrir de manière surprenante qu'en utilisant des dispersions aqueuses à base de céramides et/ou glycocéramides associés à des agents tensio-actifs cationiques particuliers, on améliorait sensiblement le démêlage des cheveux sans les alourdir ni les graisser, tout en obtenant un lissage et un gainage réguliers de la racine à la pointe des cheveux.

Les dispersions, selon l'invention, permettent également de diminuer la mouillabilité des cheveux et donc d'obtenir un séchage plus rapide.

Les dispersions cationiques selon l'invention sont particulièrement appropriées pour le traitement des cheveux sensibilisés et des cheveux fins. Elles sont particulièrement stables.

La demanderesse a constaté également que les dispersions cationiques de l'invention présentaient des propriétés cosmétiques vis-à-vis de la peau tout à fait satisfaisantes et pouvaient être appliquées pour les traitements et le soin de la peau.

La présente invention a pour objet une dispersion cationique à base de céramides et/ou de glycocéramides associés à des agents tensio-actifs cationiques particuliers.

Un autre objet concerne des compositions cosmétiques pour le traitement des cheveux ou de la peau renfermant ces dispersions.

On appelle "traitement cosmétique" un traitement ayant pour effet d'améliorer l'aspect esthétique des cheveux ou de la peau.

Un autre objet concerne des procédés de traitements cosmétiques utilisant les compositions de l'invention.

D'autres objets de l'invention apparaîtront à la lumière de la description et des exemples qui suivent.

Les dispersions cationiques de l'invention sont caractérisées par le fait qu'eues contiennent dans un milieu aqueux :

1) au moins un céramide ou glycocéramide ou un mélange de céramides et/ou de glycocéramides naturel(s) ou synthétique(s) de formule :

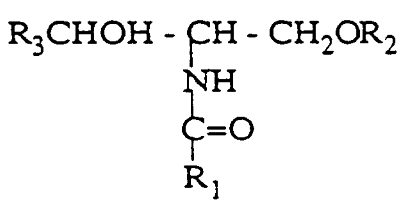

(I)

dans laquelle :

$R_1$ désigne un radical alkyle, linéaire ou ramifié, saturé ou insaturé, dérivé d'acides gras en $C_{14}$-$C_{30}$, ledit radical pouvant être substitué par un groupement hydroxyle en position $\alpha$ ou un groupement hydroxyle en position $\omega$ estérifié par un acide gras saturé ou insaturé en $C_{16}$-$C_{30}$;

$R_2$ désigne un hydrogène ou un radical (glycosyle)$_n$, -(galactosyle)$_m$ ou sulfogalactosyle, où

n est un entier variant de 1 à 4, et

m est un entier variant de 1 à 8;

$R_3$ désigne un radical hydrocarboné en $C_{15}$-$C_{26}$, saturé ou insaturé en position $\alpha$, pouvant être substitué

par un ou plusieurs radicaux alkyle en $C_1$-$C_{14}$; dans le cas des céramides ou glycéramides naturels, $R_3$ peut également désigner un radical α-hydroxyalkyle en $C_{15}$-$C_{26}$, le groupement hydroxyle étant éventuellement estérifié par α-hydroxy acide en $C_{16}$-$C_{30}$; et

2) au moins un agent tensio-actif cationique de formule :

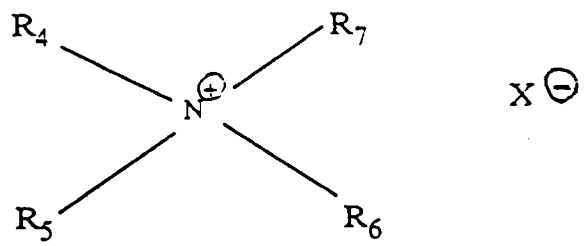

(II)

dans laquelle X désigne un anion et :

a) $R_4$, $R_5$ et $R_6$, identiques ou différents, désignent un radical alkyle en $C_1$-$C_4$; $R_7$ désigne un radical alkyle en $C_{22}$;

ou b) $R_4$ et $R_5$ sont des radicaux alkyle en $C_1$-$C_4$, identiques ou différents; et

(i) $R_6$ et $R_7$ sont des radicaux alkyle en $C_{10}$-$C_{22}$, identiques ou différents, sous réserve que le nombre total d'atomes de carbone de $R_6$ et $R_7$ soit supérieur ou égal à 20; le radical alkyle pouvant être interrompu par un groupement ester et/ou un groupement amido; ou

(ii) le radical $R_7$ désigne un groupement benzyle et $R_6$ un radical alkyle en $C_{22}$; ou

c) $R_4$ désigne un radical alkyle en $C_1$-$C_4$;

$R_5$ désigne un radical (alkyl et/ou alcényl)amidoéthyle, dans lequel le radical alkyle et/ou alcényle est en $C_{13}$-$C_{21}$;

$R_6$ et $R_7$ forment ensemble avec l'azote auquel ils sont liés un hétérocycle 4,5-dihydroimidazole substitué en position 2, par un radical alkyle et/ou alcényle en $C_{13}$-$C_{21}$.

Dans la formule (II) telle que définie ci-dessus, l'anion X désigne de préférence le chlore ou $CH_3OSO_3$, et $R_4$ désigne de préférence méthyle.

Le rapport en poids céramides et/ou glycocéramides/agents tensio-actifs cationiques, est de préférence inférieur ou égal à 2.

Les céramides et/ou glycocéramides de formule (I) sont utilisés, seuls ou en mélanges. Ils sont préparés à partir d'extraits naturels issus de la peau de porc, du cerveau de boeuf, de l'oeuf, des cellules du sang, des plantes. etc. Ils sont décrits dans les brevets JA-86/260008 et JA-87/120308 ainsi que dans la demande EP-0278505.

Parmi les composés de formule (I) telle que définie ci-dessus, on utilise de préférence ceux pour lesquels :

$R_1$ désigne un alkyle saturé ou insaturé, dérivé d'acide gras en $C_{16}$-$C_{22}$;

$R_2$ désigne hydrogène;

$R_3$ désigne un radical linéaire saturé en $C_{15}$.

De tels composés sont par exemple :

la N-linoléoyldihydrosphingosine

la N-oléoyldihydrosphingosine

la N-palmitoyldihydrosphingosine

la N-stéaroyldihydrosphingosine

la N-béhénoyldihydrosphingosine.

ou les mélanges de ces composés.

On utilise également de préférence ceux pour lesquels :

$R_1$ désigne un radical alkyle saturé ou insaturé, dérivé d'acide gras;

$R_2$ désigne galactosyle ou sulfogalactosyle; et

$R_3$ désigne $-CH=CH-(CH_2)_{12}-CH_3$.

On peut citer le produit constitué d'un mélange de ces composés, vendu sous la dénomination commerciale GLYCOCER par la Société WAITAKI INTERNATIONAL BIOSCIENCES.

Les agents tensio-actifs cationiques de formule (II) de l'invention, sont de préférence choisis dans le groupe formé par :

a) les halogénures de tétraalkylammonium tels que le chlorure de béhényltriméthylammonium ou le chlorure de diméthyldistéarylammonium.

b) un sel d'ammonium quaternaire de formule (III) :

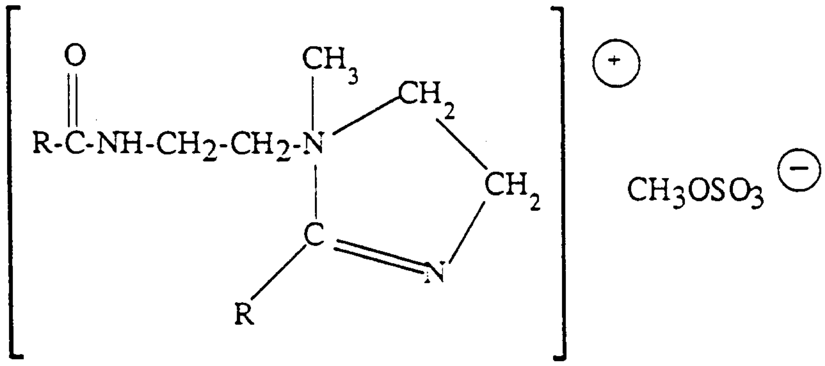

(III)

dans laquelle R désigne un mélange de radicaux alcényle et/ou alkyle en $C_{13}$-$C_{21}$ dérivé des acides gras du suif, comme par exemple les produits vendus sous la dénomination commerciale REWOQUAT (W 75, W 75 PG, W 90, W 90 DPG, W 1599, W 75 H) par la Société REWO.

c) le chlorure de stéaramidopropyldiméthyl(myristylacétate) ammonium, comme par exemple le produit vendu sous la dénomination CERAPHYL 70 par la Société MALLINCKRODT.

Les dispersions cationiques conformes à l'invention, peuvent être préparées par empâtage de l'agent tensio-actif cationique et du céramide, suivi d'une fusion du mélange à une température d'environ 80 ° C, puis d'une addition d'eau chaude (80-90 ° C) sous agitation vive à l'ultraturrax.

Dans les dispersions selon l'invention, le composé céramide et/ou glycocéramide de formule (I) est présent dans des concentrations comprises entre 0,01 et 15% en poids, de préférence entre 0,05 et 10% en poids par rapport au poids total de la dispersion et le tensio-actif cationique de formule (II) est présent dans des concentrations de 0,01 à 15% en poids et de préférence de 0,05 à 10% en poids par rapport au poids total de la dispersion.

Les dispersions cationiques selon l'invention peuvent être incorporées dans des compositions cosmétiques pour le traitement des cheveux ou de la peau, pour être utilisées en particulier comme shampooing, comme produits à rincer appliqués avant ou après shampooing, avant ou après coloration ou décoloration, avant ou après permanente ou défrisage ou entre leurs deux étapes de réduction et d'oxydation; comme produits capillaires de soin non rincés, à appliquer après un shampooing; comme lotions de mise en plis ou de brushing; comme compositions pour le soin pour la peau.

Ces compositions cosmétiques contiennent alors les céramides et/ou glycocéramides de formule (I) dans des proportions comprises entre 0,005 et 15% en poids et de préférence entre 0,01 et 10% par rapport au poids total de la composition et l'agent tensio-actif cationique de formule (II) dans des proportions comprises entre 0,01 et 15% en poids et de préférence entre 0,05 et 10% en poids par rapport au poids total de la composition.

Les compositions cosmétiques selon l'invention présentent un pH généralement compris entre 2 et 9 et plus particulièrement entre 3 et 7.

Ces compositions peuvent se présenter sous forme de liquides plus ou moins épaissis, de gels, de crèmes, de mousses aérosols ou de sprays.

Les compositions peuvent contenir également en plus de la dispersion définie précédemment, des agents régulateurs de viscosité, tels que des électrolytes, des hydrotropes ou des épaississants. Parmi ces composés, on peut citer notamment : le chlorure de sodium, le xylènesulfonate de sodium, les dérivés de la cellulose, tels que par exemple la càrboxyméthylcellulose, l'hydroxypropylcellulose, les gommes de xanthane, la gomme de guar, des gommes de guar hydroxypropylées et les scléroglucanes.

Ces agents régulateurs de viscosité sont utilisés dans des proportions allant jusqu'à 15% en poids par rapport au poids total de la composition et de préférence inférieure à 6%.

Les compositions conformes à l'invention peuvent éventuellement contenir en outre d'autres agents ayant pour effet d'améliorer les propriétés cosmétiques des cheveux ou de la peau, pourvu qu'ils n'altèrent pas la stabilité des compositions, comme des polymères anioniques, non-ioniques ou cationiques ou des protéines quaternisées ou non et des silicones.

Les polymères cationiques, non-ioniques ou anioniques, les protéines quaternisées ou non et les silicones, sont utilisés dans les compositions cosmétiques de l'invention dans des proportions comprises entre 0,05 et 6% et de préférence entre 0,1 et 3% par rapport au poids total de la composition.

Les compositions selon l'invention peuvent également contenir différents adjuvants habituellement utilisés en cosmétique, tels que des parfums, des conservateurs, des séquestrants, des stabilisateurs de mousse, des agents propulseurs, des colorants, des agents acidifiants ou alcalinisants, ainsi que d'autres adjuvants selon l'usage envisagé.

Un autre objet de l'invention consiste en un procédé de traitement cosmétique des cheveux ou de la peau comprenant l'application d'une composition selon l'invention, cette application pouvant être éventuellement suivie d'un rinçage.

Les exemples qui suivent servent à illustrer l'invention sans toutefois la limiter.

EXEMPLE 1

On prépare une dispersion de composition suivante :

| | | | |
|---|---|---|---|
| - N-oléoyldihydrosphingosine de formule : | | 2 | g |

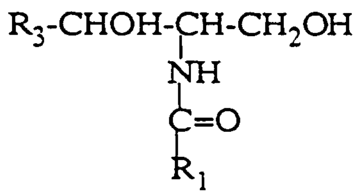

dans laquelle :

$R_3 = C_{15}H_{31}$

$R_1 = C_{17}H_{33}$

| | | | |
|---|---|---|---|
| - Chlorure de diméthyldistéarylammonium | | 2 | g |
| - Parfum, conservateur qs | | | |
| - HCl qs pH=4 | | | |
| - Eau | qsp | 100 | g |

On applique cette dispersion cationique sur des cheveux mouillés après un simple shampooing ou un shampooing faisant suite à une coloration capillaire. Après rinçage à l'eau, les cheveux mouillés sont uniformément lisses et se démêlent régulièrement bien de la racine à la pointe.

Après séchage, ils sont lisses et légers et la coiffure présente un excellent maintien.

EXEMPLE 2

On prépare une dispersion de composition suivante :

| | |
|---|---|
| - Céramide de l'exemple 1 | 0,5 g |
| - Chlorure de béhényl triméthyl ammonium à 80% de MA | 2 g MA |
| - Parfum, conservateur qs | |
| - HCl qs pH = 4 | |
| - Eau qsp | 100 g |

On applique cette dispersion cationique sur les cheveux comme à l'exemple 1, en obtenant les mêmes résultats.

EXEMPLE 3

On prépare une dispersion de composition suivante :

| | |
|---|---|
| - Céramide de l'exemple 1<br>- Sel d'ammonium quaternaire de formule (III) vendu sous la dénomination REWOQUAT W 75 PG à 75% de MA par la Société REWO<br>- Triéthanolamine qs pH = 6<br>- Parfum, conservateur qs<br>- Eau qsp | 0,5 g<br>2 g MA<br><br><br><br>100 g |

On applique cette dispersion cationique sur des cheveux ayant subi une réduction : 1ère étape d'une ondulation permanente. Après rinçage on procède à l'étape d'oxydation de la permanente. Les cheveux mouillés et séchés présentent les mêmes avantages que ceux décrits dans l'exemple 1.

EXEMPLE 4

On prépare une dispersion de composition suivante :

| | |
|---|---|
| - Chlorure de diméthyl distéaryl ammonium<br>- Céramide de l'exemple 1<br>- Alcool stéarylique<br>- Alcool cétylique<br>- Mélange d'alcool cétylstéarylique et d'alcool cétylstéarylique oxyéthyléné à 33 moles d'oxyde d'éthylène<br>- Triéthanolamine qs pH = 7<br>- Eau qsp | 0,7 g<br>1,4 g<br>1,4 g<br>1,4 g<br>3,6 g<br><br><br>100 g |

On applique cette dispersion cationique sur des cheveux mouillés ayant subi une ondulation permanente, c'est-à-dire après la phase d'oxydation. Après rinçage à l'eau, les cheveux mouillés et séchés présentent les mêmes avantages qu'à l'exemple 1.

EXEMPLE 5

On prépare une dispersion de composition suivante :

| | |
|---|---|
| - Céramide de l'exemple 1<br>- Chlorure de stéaramidopropyl diméthyl (myristylacétate)ammonium vendu sous la dénomination CERAPHYL 70 par la Société MALLINCKRODT<br>- pH spontané = 5<br>- Eau qsp | 2 g<br>2 g<br><br><br>100 g |

On applique cette dispersion cationique sur des cheveux ayant subi une décoloration. Après rinçage, les cheveux mouillés et séchés présentent les mêmes avantages qu'a l'exemple 1.

EXEMPLE 6

On prépare une dispersion de composition suivante :

- N-béhénoyl dihydrosphingosine de formule : 0,5 g

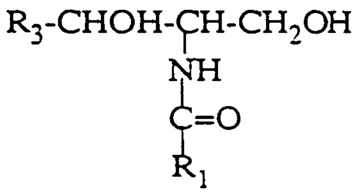

dans laquelle :

$R_1 = C_{21}H_{43}$

$R_3 = C_{15}H_{31}$

- Chlorure de diméthyl distéaryl ammonium 5 g
- Conservateur qs
- pH spontané = 5
- Eau qsp 100 g

On applique cette dispersion cationique sur des cheveux mouillés, après shampooing. Sans rincer les cheveux, on les sèche puis on les coiffe. Les cheveux sont uniformément lisses, légers, gainés et faciles à démêler de la racine à la pointe.

EXEMPLE 7

On prépare une dispersion de composition suivante :

| | |
|---|---|
| - Glycocéramide vendu à 42% de MA sous la dénomination GLYCOCER par la Société WAITAKI INTERNATIONAL BIOSCIENCES | 0,1 g MA |
| - Hydrolysat de collagène quaternisé par du cocoylamidopropyl diméthylamine, vendu à 30% de MA sous la dénomination LEXEIN QX 3000 par la Société AQUALON | 0,3 g MA |
| - Sel d'ammonium quaternaire de formule (III), vendu à 75% de MA sous la dénomination REWOQUAT W 75 PG par la Société REWO | 0,3 g MA |
| - Hydroxyéthylcellulose | 0,4 g |
| - Conservateur qs | |
| - pH spontané = 5 | |
| - Eau qsp | 100 g |

On applique cette dispersion cationique sur des cheveux lavés et mouillés, éventuellement enroulés sur des bigoudis. Sans rincer les cheveux, on les sèche. Les cheveux présentent les mêmes avantages qu'à l'exemple 6.

EXEMPLE 8

On prépare une dispersion de composition suivante :

| | |
|---|---|
| - Céramide de l'exemple 1 | 0,5 g |
| - Chlorure de diméthyl distéaryl ammonium | 5 g |
| - Polydiméthylsiloxane vendu sous la dénomination SILBIONE huile 47 V 50 par la Société RHONE POULENC | 0:1 g |
| - Conservateur qs | |
| - pH spontané = 5 | |
| - Eau qsp | 100 g |

On applique cette dispersion cationique sur des cheveux propres et mouillés. Après rinçage à l'eau, les cheveux mouillés et séchés présentent les mêmes avantages qu'à l'exemple 1.

EXEMPLE 9

On prépare une dispersion de composition suivante :

- N-béhénoyldihydrosphingosine de formule : 0,01 g

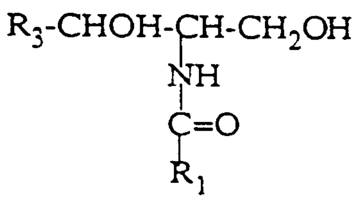

dans laquelle :

$R_1 = C_{21}H_{43}$

$R_3 = C_{15}H_{31}$

- Sel d'ammonium quaternaire de formule (III), vendu à 75% de MA sous la dénomination REWOQUAT W 75 PG par la Société REWO 0,5 g MA
- Conservateur qs
- HCl qs pH=5
- Eau qsp 100 g

On applique cette dispersion cationique sur des cheveux mouillés, après un shampooing. Sans rincer les cheveux, on les sèche puis on les coiffe. Les cheveux présentent les mêmes avantages qu'à l'exemple 6.

EXEMPLE 10

On prépare une composition pour le bain ou la douche suivante :

| | |
|---|---|
| - Glycocéramide vendu à 42% de MA sous la dénomination GLYCOCER par la Société WAITAKI INTERNATIONAL BIOSCIENCES | 0,2 g MA |
| - Chlorure de béhényltriméthylammonium | 0.63 g |
| - Alcool laurique oxyéthyléné sulfate de sodium, vendu sous la dénomination EMPICOL ESB/3FL par la Société MARCHON | 30,0 g |
| - Acide éther carboxylique polyoxyéthyléné à 10 moles d'oxyde d'éthylène, vendu sous la dénomination AKYPO RLM 100 par la Société CHEM Y, à 90% de MA | 2,3 g MA |
| - Cocoylamidopropyl-hydroxypropylsulfobétàine à 50% de MA vendue sous la dénomination AMONYL 675 SB par la Société SEPPIC | 4,8 g MA |
| - Parfum qs | |
| - Eau qsp | 100,0 g |

Ce shampooing pour le bain ou la douche présente une mousse très douce et communique à la peau de la douceur.

**Revendications**

**1.** Dispersion cationique pour le traitement et le soin des cheveux ou de la peau, caractérisée par le fait qu'elle contient dans un milieu aqueux :

1) au moins un céramide ou glycocéramide ou un mélange de céramides et/ou de glycocéramides, naturels ou synthétiques, de formule suivante :

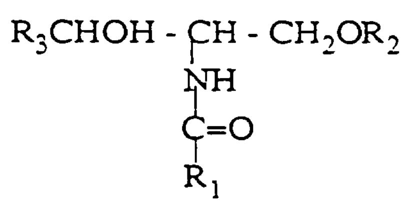

(I)

dans laquelle :

$R_1$ désigne un radical alkyle, linéaire ou ramifié, saturé ou insaturé, dérivé d'acides gras en $C_{14}$-$C_{30}$, ledit radical pouvant être substitué par un groupement hydroxyle en position $\alpha$ ou un groupement hydroxyle en position $\omega$ estérifié par un acide gras saturé ou insaturé en $C_{16}$-$C_{30}$;

$R_2$ désigne un hydrogène ou un radical (glycosyle)$_n$, -(galactosyle)$_m$ ou sulfogalactosyle, où

n est un entier variant de 1 à 4, et

m est un entier variant de 1 à 8;

$R_3$ désigne un radical hydrocarboné en $C_{15}$-$C_{26}$, saturé ou insaturé en position $\alpha$, pouvant être substitué par un ou plusieurs radicaux alkyle en $C_1$-$C_{14}$; dans le cas des céramides ou glycéramides naturels, $R_3$ peut également désigner un radical $\alpha$-hydroxyalkyle en $C_{15}$-$C_{26}$, le groupement hydroxyle étant éventuellement estérifié par $\alpha$-hydroxy acide en $C_{16}$-$C_{30}$; et

2) au moins un agent tensio-actif cationique de formule :

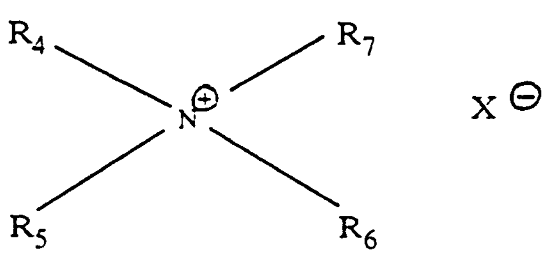

(II)

dans laquelle X désigne un anion et :

a) $R_4$, $R_5$ et $R_6$, identiques ou différents, désignent un radical alkyle en $C_1$-$C_4$; $R_7$ désigne un radical alkyle en $C_{22}$;

ou b) $R_4$ et $R_5$ sont des radicaux alkyle en $C_1$-$C_4$, identiques ou différents; et

(i) $R_6$ et $R_7$ sont des radicaux alkyle en $C_{10}$-$C_{22}$, identiques ou différents, sous réserve que le nombre total d'atomes de carbone de $R_6$ et $R_7$ soit supérieur ou égal à 20; le radical alkyle pouvant être interrompu par un groupement ester et/ou un groupement amido; ou

(ii) le radical $R_7$ désigne un groupement benzyle et $R_6$ un radical alkyle en $C_{22}$; ou

c) $R_4$ désigne un radical alkyle en $C_1$-$C_4$;

$R_5$ désigne un radical (alkyl et/ou alcényl)amidoéthyle, dans lequel le radical alkyle et/ou alcényle est en $C_{13}$-$C_{21}$;

$R_6$ et $R_7$ forment ensemble avec l'azote auquel ils sont liés un hétérocycle 4,5-dihydroimidazole substitué en position 2, par un radical alkyle et/ou alcényle en $C_{13}$-$C_{21}$.

**2.** Dispersion selon la revendication 1, caractérisée par le fait que le rapport en poids : céramide et/ou glycocéramide de formule (I)/agent tensio-actif de formule (II), est inférieur ou égal à 2.

**3.** Dispersion selon la revendication 1 ou 2, caractérisée par le fait que dans la formule (II), X désigne le chlore ou le groupe $CH_3OSO_3^-$ et $R_4$ désigne le radical méthyle.

**4.** Dispersion selon l'une quelconque des revendications 1 à 3, caractérisée par le fait que les composés céramides et/ou glycocéramides de formule (I) ou leurs mélanges, sont choisis dans le groupe formé par :

a) les composés de formule (I) pour lesquels $R_1$ désigne un radical alkyle, saturé ou insaturé, dérivé d'acide gras en $C_{16}$-$C_{22}$;

$R_2$ désigne hydrogène;

$R_3$ désigne un radical hydrocarboné linéaire saturé en $C_{15}$;

ainsi que leurs mélanges;

b) les composés de formule (I) pour lesquels $R_1$ désigne un radical alkyle saturé ou insaturé, dérivé d'acide gras; $R_2$ désigne galactosyle ou sulfogalactosyle; et $R_3$ désigne le groupe $-CH=CH-(CH_2)_{12}-CH_3$; ainsi que leurs mélanges.

**5.** Dispersion selon l'une quelconque des revendications 1 à 4, caractérisée par le fait que l'agent tensio-actif cationique, de formule (II), est choisi dans le groupe formé par :

a) les halogénures de tétraalkylammonium tels que le chlorure de béhényltriméthylammonium ou le chlorure de diméthyldistéarylammonium.

b) un sel d'ammonium quaternaire de formule :

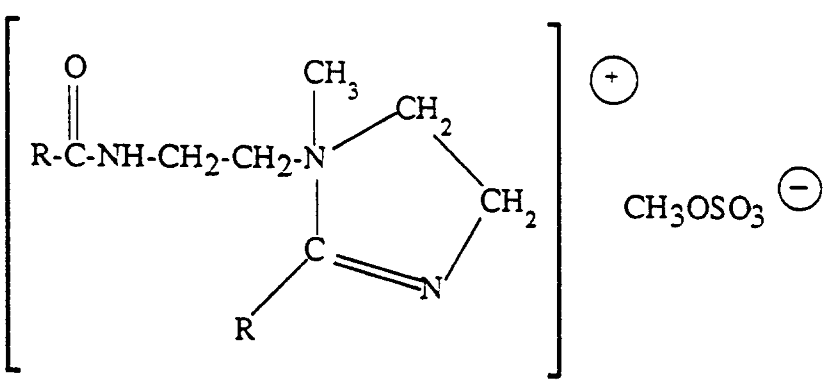

(III)

dans laquelle R désigne un mélange de radicaux alcényle et/ou alkyle en $C_{13}$-$C_{21}$ dérivé des acides gras du suif;

c) le chlorure de stéaramidopropyldiméthyl(myristylacétate) ammonium.

**6.** Dispersion selon l'une quelconque des revendications 1 à 5, caractérisée par le fait que le composé de formule (I) est présent dans des concentrations comprises entre 0,01 et 15% en poids par rapport au

poids total de la dispersion et que l'agent tensio-actif cationique de formule (II) est présent dans des concentrations comprises entre 0,01 et 15% en poids.

**7.** Composition cosmétique de traitement des cheveux ou de la peau, caractérisée par le fait qu'elle renferme dans un support aqueux cosmétiquement acceptable, au moins une dispersion définie selon l'une quelconque des revendications 1 à 6.

**8.** Composition selon la revendication 7, caractérisée par le fait qu'elle contient le composé céramide et/ou glycocéramide de formule (I) dans une concentration comprise entre 0,005 et 15% en poids et l'agent tensio-actif cationique de formule (II) dans des concentrations comprises entre 0,01 et 15% en poids par rapport au poids total de la composition.

**9.** Composition selon la revendication 7 ou 8, caractérisée par le fait qu'elle se présente sous forme de liquides plus ou moins épaissis, de gels, de crèmes, de mousses aérosols ou de sprays.

**10.** Composition selon l'une quelconque des revendications 7 à 9, caractérisée par le fait qu'elle contient, en plus, des agents régulateurs de viscosité dans des proportions allant jusqu'à 15% en poids par rapport au poids total de la composition.

**11.** Composition selon l'une quelconque des revendications 7 à 10, caractérisée par le fait qu'elle contient des agents de conditionnement des cheveux ou de la peau choisis parmi les polymères anioniques, non-ioniques ou cationiques, des protéines quaternisées ou non, des silicones, qui n'altèrent pas la stabilité de la composition, dans des proportions comprises entre 0,05 et 6% en poids par rapport au poids total de la composition.

**12.** Composition selon l'une quelconque des revendications 7 à 11, caractérisée par le fait qu'eue contient, en plus, des parfums, des conservateurs, des séquestrants, des stabilisateurs de mousse, des agents propulseurs, des colorants, des agents acidifiants ou alcalinisants ou autres adjuvants actuellement utilisés en cosmétique.

**13.** Utilisation cosmétique de la composition selon l'une quelconque des revendications 7 à 12,comme shampooing, comme produit capillaire à rincer, à appliquer avant ou après shampooing, avant ou après coloration ou décoloration, avant ou après permanente ou défrisage ou entre leurs deux étapes de réduction et d'oxydation; comme produit capillaire de soin non rincé à appliquer après un shampooing; comme lotion de mise en plis ou de brushing; comme produit pour le soin de la peau.

**14.** Procédé de traitement cosmétique des cheveux ou de la peau, caractérisé par le fait qu'il comprend une application d'une composition selon l'une quelconque des revendications 7 à 13, suivie éventuellement d'un rinçage.

## Claims

**1.** Cationic dispersion for the treatment and care of the hair and the skin, characterized in that it contains in an aqueous medium:

1) at least one natural or synthetic ceramide or glycoceramide, or a mixture of natural or synthetic ceramides and/or glycoceramides of the following formula:

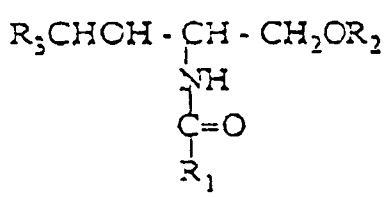

(I)

in which:

$R_1$ denotes a saturated or unsaturated, linear or branched alkyl radical derived from $C_{14}$-$C_{30}$ fatty acids, it being possible for the said radical to be substituted by a hydroxyl group in the $\alpha$ position or

a hydroxyl group in the $\omega$ position esterified by a saturated or unsaturated, $C_{16}$-$C_{30}$ fatty acid;
$R_2$ denotes a hydrogen or a $(glycosyl)_n$, $-(galactosyl)_m$ or sulfogalactosyl radical, where
n is an integer ranging from 1 to 4, and
m is an integer ranging from 1 to 8;
$R_3$ denotes a $C_{15}$-$C_{26}$ hydrocarbon radical, saturated or unsaturated in the $\alpha$ position, which can be substituted by one or a number of $C_1$-$C_{14}$ alkyl radicals; in the case of natural ceramides or glyceramides [sic], $R_3$ can also denote a $C_{15}$-$C_{26}$ $\alpha$-hydroxyalkyl radical, the hydroxyl group optionally being esterified by a $C_{16}$-$C_{30}$ $\alpha$-hydroxy acid; and
2) at least one cationic surface-active agent of formula:

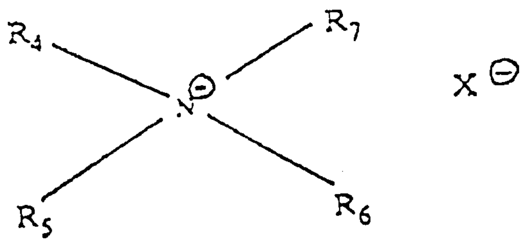

(II)

in which X denotes an anion and:

a) $R_4$, $R_5$ and $R_6$, which are identical or different, denote a $C_1$-$C_4$ alkyl radical; $R_7$ denotes a $C_{22}$ alkyl radical;

or b) $R_4$ and $R_5$ are $C_1$-$C_4$ alkyl radicals, which are identical or different; and

(i) $R_6$ and $R_7$ are $C_{10}$-$C_{22}$ alkyl radicals, which are identical or different, with the proviso that the total number of carbon atoms of $R_6$ and $R_7$ is greater than or equal to 20; it being possible for the alkyl radical to be interrupted by an ester group and/or an amido group; or

(ii) the radical $R_7$ denotes a benzyl group and $R_6$ a $C_{22}$ alkyl radical; or

c) $R_4$ denotes a $C_1$-$C_4$ alkyl radical;

$R_5$ denotes an (alkyl and/or alkenyl)amidoethyl radical, in which the alkyl and/or alkenyl radical is $C_{13}$-$C_{21}$;

$R_6$ and $R_7$ form, together with the nitrogen to which they are bonded, a 4,5-dihydroimidazole heterocycle substituted in position 2 by a $C_{13}$-$C_{21}$ alkyl and/or alkenyl radical.

**2.** Dispersion according to claim 1, characterized in that the ratio by weight: ceramide and/or glycoceramide of formula (I)/surface-active agent of formula (II), is less than or equal to 2.

**3.** Dispersion according to Claim 1 or 2, characterized in that in the formula (II), X denotes chlorine or the $CH_3OSO_3^-$ group and $R_4$ denotes the methyl radical.

**4.** Dispersion according to any one of Claims 1 to 3, characterized in that the ceramide and/or glycoceramide compounds of formula (I) or their mixtures are chosen from the group formed by:

a) the compounds of formula (I) for which $R_1$ denotes a saturated or unsaturated alkyl radical derived from a $C_{16}$-$C_{22}$ fatty acid;
$R_2$ denotes hydrogen;
$R_3$ denotes a saturated linear $C_{15}$ hydrocarbon radical;
and their mixtures;

b) the compounds of formula (I) for which $R_1$ denotes a saturated or unsaturated alkyl radical derived from a fatty acid; $R_2$ denotes galactosyl or sulfogalactosyl; and $R_3$ denotes the group $-CH=CH-(CH_2)_{12}-CH_3$; and their mixtures.

**5.** Dispersion according to any one of Claims 1 to 4, characterized in that the cationic surface-active agent, of formula (II), is chosen from the group formed by:

a) the tetraalkylammonium halides, such as behenyltrimethylammonium chloride or dimethyldistearylammonium chloride.

b) a quaternary ammonium salt of formula:

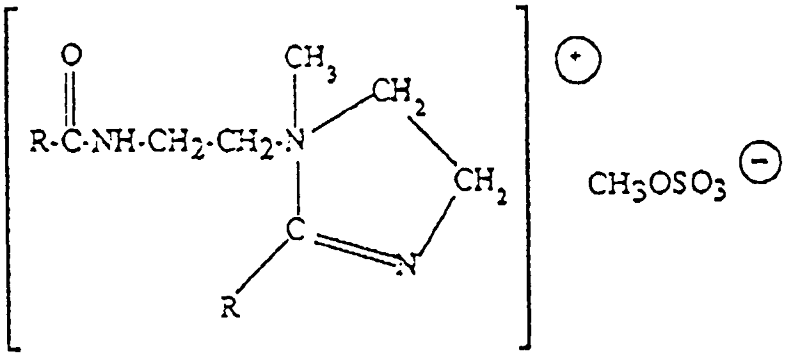

(III)

in which R denotes a mixture of $C_{13}$-$C_{21}$ alkenyl and/or alkyl radicals derived from tallow fatty acids,
c) stearamidopropyldimethyl(myristylacetate)ammonium chloride.

**6.** Dispersion according to any one of Claims 1 to 5, characterized in that the compound of formula (I) is present in concentrations between 0.01 and 15% by weight with respect to the total weight of the dispersion and in that the cationic surface-active agent of formula (II) is present in concentrations between 0.01 and 15% by weight.

**7.** Cosmetic composition for treating the hair or the skin, characterized in that it contains, in a cosmetically acceptable aqueous vehicle, at least one dispersion defined according to any one of Claims 1 to 6.

**8.** Composition according to claim 7, characterized in that it contains the ceramide and/or glycoceramide compound of formula (I) in a concentration between 0.005 and 15% by weight and the cationic surface-active agent of formula (II) in concentrations between 0.01 and 15% by weight with respect to the total weight of the composition.

**9.** Composition according to Claim 7 or 8, characterized in that it is provided in the form of more or less thick liquids, of gels, of creams, of aerosol foams or of sprays.

**10.** Composition according to any one of Claims 7 to 9, characterized in that it additionally contains viscosity regulating agents in proportions ranging up to 15% by weight with respect to the total weight of the composition.

**11.** Composition according to any one of Claims 7 to 10, characterized in that it contains hair or skin conditioning agents chosen from anionic, nonionic or cationic polymers, quaternized or non-quaternized proteins or silicones, which do not detrimentally affect the stability of the composition, in proportions between 0.05 and 6% by weight with respect to the total weight of the composition.

**12.** Composition according to any one of Claims 7 to 11, characterized in that it additionally contains fragrances, preserving agents, sequestering agents, foam stabilizing agents, propellants, dyes, acidifying or basifying agents or other adjuvants currently used in cosmetics.

**13.** Cosmetic use of the composition according to any one of Claims 7 to 12, as a shampoo or a rinsable hair product, to be applied before or after shampooing, before or after dyeing or bleaching, before or after a permanent wave or hair straightening or between their two stages of reduction and oxidation ; as a non-rinsed hair care product, to be applied after a shampoo ; as a hair setting or blow drying lotion ; or as a skin care product.

**14.** Process for the cosmetic treatment of the hair or the skin , characterized in that it comprises an application of a composition according to any one of Claims 7 to 13, optionally followed by rinsing.

**Patentansprüche**

1. Kationische Dispersion zur Behandlung und Pflege der Haare oder der Haut, dadurch **gekennzeichnet**, daß sie in einem wässrigen Milieu enthält:

1) mindestens ein natürliches oder synthetisches Ceramid oder Glycoceramid oder eine Mischung der Ceramide und/oder Glycoceramide der Formel:

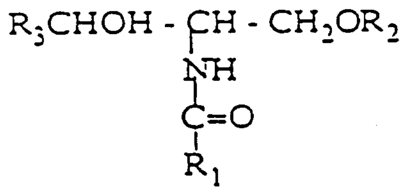

(I)

worin gilt:

$R_1$ bedeutet einen linearen oder verzweigten gesättigten oder ungesättigten Alkylrest, der von $C_{14-30}$-Fettsäuren abgeleitet ist, wobei der genannte Rest mit einer Hydroxylgruppe in Positon $\alpha$ oder Hydroxylgruppe in Position $\omega$ substituiert sein kann, welche mit einer gesättigten oder ungesättigten $C_{16-30}$-Fettsäure verestert ist;

$R_2$ bedeutet Wasserstoff oder einen $(Glycosyl)_n$-,$(Galactosyl)_m$- oder Sulfogalactosyl-Rest, worin n eine ganze Zahl von 1 bis 4 und m eine ganze Zahl von 1 bis 8 sind;

$R_3$ bedeutet einen gesättigten oder in Position $\alpha$ ungesättigten $C_{15-28}$-Kohlenwasserstoffrest, der mit einem oder mehreren $C_{1-14}$-Alkylresten substituiert sein kann; im Falle natürlicher Ceramide oder Glycoceramide kann $R_3$ auch einen $C_{15-26}$-$\alpha$-Hydroxyalkylrest bedeuten, dessen Hydroxylgruppe gegebenenfalls mit einer $C_{16-30}$-$\alpha$-Hydroxysäure verestert ist, sowie

2) mindestens ein kationisches oberflächenaktives Mittel der Formel:

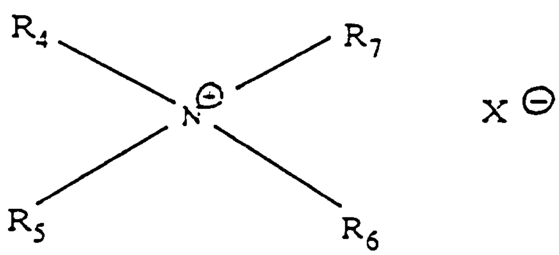

(II)

worin X ein Anion bedeutet und ferner gilt:

a) $R_4$, $R_5$ und $R_6$ bedeuten, gleich oder verschieden einen $C_{1-4}$-Alkylrest; $R_7$ bedeutet einen $C_{22}$-Alkylrest; oder

b) $R_4$ und $R_5$ sind gleiche oder verschiedene $C_{1-4}$-Alkylreste; und

(i) $R_6$ und $R_7$ sind gleiche oder verschiedene $C_{10-22}$-Alkylreste, mit der Maßgabe, daß die Gesamtzahl der Kohlenstoffatome von $R_6$ und $R_7$ mehr als oder gleich 20 ist, wobei der Alkylrest durch eine Ester- und/oder Amidgruppe unterbrochen sein kann; oder

(ii) der Rest $R_7$ bedeuten eine Benzylgruppe und $R_6$ einen $C_{22}$-Alkylrest; oder

c) $R_4$ bedeutet einen $C_{1-4}$-Alkylrest;

$R_5$ bedeutet einen Alkyl- und/oder Alkenylamidoethylrest, worin der Alkyl- und/oder Alkenylrest ein $C_{13-21}$-Rest ist;

$R_6$ und $R_7$ bilden zusammen mit dem Stickstoff, an den sie gebundens sind, einen 4,5-Dihydroimidazol-Heterozyklus, der in Position 2 mit einem $C_{13-21}$-Alkyl- und/oder -Alkenylrest substituiert ist.

**2.** Dispersion gemäß Anspruch 1,
dadurch **gekennzeichnet**, daß
das Gewichtsverhältnis von Ceramid und/oder Glycoceramid der Formel (I) zum oberflächenaktiven Mittel der Formel (II) weniger als oder gleich 2 beträgt.

**3.** Dispersion gemäß Anspruch 1 oder 2,
dadurch **gekennzeichnet**, daß
in der Formel (II) X Chlorid oder die Gruppe $CH_3OSO_3^-$ und $R_4$ den Methylrest bedeuten.

**4.** Dispersion gemäß jedem der Ansprüche 1 bis 3,
dadurch **gekennzeichnet**, daß
die Ceramid- und/oder Glycoceramidverbindungen der Formel (I) oder deren Mischungen ausgewählt sind aus der Gruppe aus:
a) Verbindungen der Formel (I), für welche $R_1$ einen gesättigten oder ungesättigten, von $C_{16-22}$-Fettsäuren abgeleiteten Alkylrest,
$R_2$ Wasserstoff und
$R_3$ einen linearen gesättigten $C_{15}$-Kohlenwasserstoffrestbedeuten, sowie aus deren Mischungen;
b) Verbindungen der Formel (I), für welche $R_1$ einen gesättigten oder ungesättigten, von Fettsäuren abgeleiteten Alkylrest, $R_2$ den Galactosyl- oder Sulfogalactosylrest und $R_3$ die Gruppe $-CH=CH-(CH_2)_{12}-CH_3$ bedeuten, sowie aus deren Mischungen.

**5.** Dispersion gemäß jedem der Ansprüche 1 bis 4,
dadurch **gekennzeichnet**, daß
das kationische oberflächenaktive Mittel der Formel (II) ausgewählt ist aus der Gruppe aus:
a) Tetraalkylammoniumhalogeniden wie dem Behenyltrimethylammonium- oder Dimethyldistearylammoniumchlorid,
b) einem quaternären Anmoniumsalz der Formel:

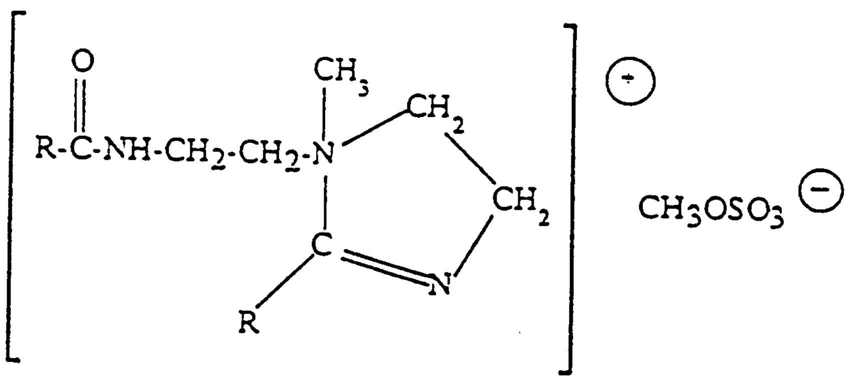

(III)

in welcher R eine Mischung aus von Talgfettsäuren abgeleiteten $C_{13-21}$-Alkenyl- und/oder -Alkylresten bedeutet, und
c) dem Stearamidopropyldimethyl(myristylacetat)ammoniumchlorid.

**6.** Dispersion gemäß jedem der Ansprüche 1 bis 5,
dadurch **gekennzeichnet**, daß
die Verbindung der Formel (I) in Konzentrationen von 0,1 bis 15 Gew.%, bezogen auf das Gesamtgewicht der Dispersion, und das kationische oberflächenaktive Mittel der Formel (II) in Konzentrationen von 0,01 bis 15 Gew.% vorhanden sind.

**7.** Kosmetische Zusammensetzung zur Behandlung der Haare oder der Haut, dadurch **gekennzeichnet**, daß sie in einem kosmetisch geeigneten wässrigen Träger mindestens eine Dispersion gemäß jedem der Ansprüche 1 bis 6 enthält.

**8.** Zusammensetzung gemäß Ansrpuch 7,
dadurch **gekennzeichnet**, daß

sie die Ceramid- und/oder Glycoceramid-Verbindung der Formel (I) in einer Konzentration von 0,005 bis 15 Gew.% und das kationische oberflächenaktive Mittel der Formel (II) in Konzentrationen von 0,01 bis 15 Gew.% enthält, bezogen auf das Gesamtgewicht der Zusammensetzung.

**9.** Zusammensetzung gemäß Anspruch 7 oder 8,
dadurch **gekennzeichnet**, daß
sie in Form mehr oder weniger verdickter Flüssigkeiten, von Gelen, Creme-Produkten, Aerosol-Schäumen oder von Spray-Produkten vorliegt.

**10.** Zusammensetzung gemäß jedem der Ansprüche 7 bis 9,
dadurch **gekennzeichnet**, daß
sie, zusätzlich, Viskositätsreguliermittel in Mengenanteilen bis zu 15 Gew.% enthält, bezogen auf das Gesamtgewicht der Zusammensetzung.

**11.** Zusammensetzung gemäß jedem der Ansprüche 7 bis 10,
dadurch **gekennzeichnet**, daß
sie Konditioniermittel für Haare oder Haut, die aus anionischen, nicht-ionischen oder kationischen Polymeren, quaternierten oder nicht quaternierten Proteinen und aus Silikonen ausgewählt sind, welche die Stabilität der Zusammensetzung nicht verändern, in Mengenanteilen von 0,05 bis 6 Gew.% enthält, bezogen auf das Gesamtgewicht der Zusammensetzung.

**12.** Zusammensetzung gemäß jedem der Ansprüche 7 bis 11,
dadurch **gekennzeichnet**, daß
sie, zusätzlich, Parfüm-Produkte, Konservierungsstoffe, Sequestriermittel, Schaumstabilisatoren, Treibmittel, Färbemittel, sauer oder alkalisch stellende Mittel oder weitere, in der Kosmetik wirksam verwendete Hilfsstoffe enthält.

**13.** Kosmetische Verwendung der Zusammensetzung gemäß jedem der Ansprüche 7 bis 12 als Shampoo, als Produkt für das Haar zur Spülung, zur Aufbringung vor oder nach einer Schamponierung, vor oder nach einer Färbung oder Entfärbung, vor oder nach einer Dauerwelle oder einem Entfrisiervorgang oder zwischen deren beiden Stufen einer Reduktions- und Oxidationsbehandlung, als Haarpflegeprodukt, das nicht zur Spülung vorgesehen ist und nach einem Schamponiervorgang aufgetragen wird, als Lotion zur Wellengebung oder zum Bürsten sowie als Produkt zur Pflege der Haut.

**14.** Kosmetisches Verfahren zur Behandlung der Haare oder der Haut,
dadurch **gekennzeichnet**, daß
man eine Zusammensetzung gemäß jedem der Ansprüche 7 bis 13 aufbringt und dann gegebenenfalls spült.